# EUROPEAN PATENT APPLICATION

(11) **EP 4 678 151 A1**
(43) Date of publication of application: **14.01.2026**
(21) Application number: 25171778.1
(22) Date of filing: 22.04.2025
(51) Int. Cl.: A61F 13/15, A61F 13/53, A61F 13/531, D04H 1/00, D04H 1/425

(54) **METHOD AND APPARATUS FOR FORMING SEPARATED FIBROUS STRUCTURES**

(30) Priority: 12.07.2024 EP 24188478
(71) Applicant: COAX Technologies S.r.l., 26010 Monte Cremasco Cremona (IT)
(72) Inventor: ZAMPOLLO, Fabio, 26013 Crema (IT)
(74) Representative: Plischke, Manfred

(57) **Abstract**

The present invention is an equipment for and a process of manufacturing of a series of separated fibrous sections of the air-laying type as well as forming laminates comprising such series of fibrous web sections. Such structures may suitably be absorbent, and may be used for or as bed-pads or for other articles, such as absorbent articles.

## Description

### Field of the invention

The present invention relates to the equipment for and the process of manufacturing a series of separated fibrous web sections of the air-laying type as well as forming laminates comprising such series of fibrous web sections. Such structures may suitably be absorbent, and may be used for or as bed-pads or for other articles, such as absorbent articles.

### Background

Fibrous sheet materials, particularly fibrous sheet materials for absorbing fluids, are manufactured for many uses, for example they are incorporated into absorbent articles such as disposable diapers, incontinent bed-pads and catamenial napkins as fluid absorption or fluid transmission and/or diffusion elements, for example, as absorbent cores that are intended to absorb and retain body fluids.

Dry laying and, more specifically, air laying processes are widely used to produce webs from dry fibers, which can in turn be used e.g., as sheet materials for absorbing fluids. Particularly, the air laying process refers to the formation of webs with a random fiber orientation. The fibrous sheet materials produced by air laying processes are soft, flexible and porous, and are particularly suitable for use as liquid absorbent structures in absorbent articles, such as disposable diapers, sanitary napkins, pantiliners, incontinent or bed-pads, and wipes. Air laying processes are well known in the art, as are improvements aiming at evenly distributing the fibers in the airlaid structure and/or enhancing processability.

In US5527171 (Niro) the deposition of air suspended fibers in homogeneous layers is described by using multiple rows of rotating impellers in a forming head.

Similarly, US6233787 (Dan-Web / Advanced Nonwoven S/A) shows an apparatus for uniformly distributing a disintegrated material on a fiber layer forming surface by a stirrer having impellers rotating a short distance above a collecting surface.

US20030070262 (Oerlikon) describes two air-lay stations placed one after the other serving for the dry production of a non-woven fiber web, the stations including a fiber feed duct, an air laying forming head, a perforated screen as collecting surface, and a suction box for supporting the deposition of the fibers on the screen. A separate fiber source is part of each station.

Further, WO200004232 (M&J) describes the production of a nonwoven web of fibers out of a fibrous material, such as cellulose pulp. The fibrous material is disintegrated by a hammer mill and deposited at the outlet of a forming head on an endless collection wire, thereby forming a web of fibers. Nits are extracted from the forming head via a transport fan and a second air duct. Furthermore, the nits are defibrated and returned to the forming head.

Also, WO2004106604A1 (Oerlikon) aims at avoiding nits by employing a fiber distributor in a forming head at optimizing the rotational speed of the wings of the distributor.

Such production units are typically operated in an "off-line" modus, i.e., the webs produced thereon are prepared at a wide web width for interim storage in roll- or spool-form, or boxed ("festooned") and further transported to a converting place where these are fed into a process for forming articles, such as wipes, bed-pads and the like, including the cutting to size.

Such an approach induces several areas for improvement:
- homogeneity of the web is always a concern and gets more relevant when lower basis weights are aimed at.
- the fibers should be individualized as much as possible, i.e., the fibers should have little or preferably no connection to other fibers when they are laid down to form the web. Thus, it would be advantageous to obtain maximum disintegration of the fibers as supplied and to maintain the fiber disintegration along the process by avoiding aggregation of fibers or forming of nits - but all this with minimized - or even no - damage to the fibers, such as breaking up fibers, and low energy input.
- as the interim handling of the webs between web forming and web converting induces significant effort, it would be advantageous to skip such a process step, and feed the freshly formed web or sections thereof directly into a connected converting step, such as sandwiching the separated webs between envelope webs, such as topsheet and backsheet, thereby forming finished articles.

### Summary (marked up vs. claims as below)

In a first aspect, the present invention is an equipment for forming a series of separated fibrous sections, exhibiting a machine (x-)direction , a cross (y-) direction and a height (z-)direction and comprising
- a first fibrous material supply and optionally a second fibrous material supply;
- a collection system comprising a collection surface comprising a plurality of air permeable regions and blocked regions , which are essentially non-permeable to air, selected from the group consisting of
   - a collection belt comprising belt guide rolls for operating the collection belt in a continuous loop and defining the machine direction;
      and
   - a pair of counterrotating vacuum drums forming a gap therebetween;
- a first forming head comprising a first forming box for depositing first fibrous material from the first fibrous material supply in a first deposition region onto the air permeable regions of the collection system;
- a first compaction unit;
- optionally a particulate material application system with a particulate material supply;
- optionally a second forming head for depositing second fibrous material from the second fibrous material supply onto the air permeable regions of the collection system;
- a first suction unit connected to the collection system to cooperate with the first forming head, optionally a second suction unit to cooperate with the second forming head, and an optionally further suction unit to cooperate with the further particulate material supply;
- a first transfer unit,
characterized in that the equipment further comprises
- a collection system comprising a collection surface comprising a plurality or air permeable regions and blocked regions, which are essentially non-permeable to air, selected from the group consisting of
   - a collection belt comprising belt guide rolls for operating the collection belt (1510) in a continuous loop and defining the machine direction,
      and
   - a pair of counterrotating vacuum drums forming a gap therebetween.

The collection belt of the collection system may exhibit an essentially constant thickness across its air permeable regions and blocked regions .

In another aspect, the present invention is a process for forming a series of separated fibrous sections, comprising the steps of
A1 - providing web forming equipment exhibiting a machine (x-)direction, a cross (y-) direction and a height (z-)direction and comprising:
   - a first fibrous material supply and optionally a second fibrous material supply;
   - a collection system, comprising a collection surface comprising a plurality of air permeable regions and blocked regions which are preferably non-permeable to air, selected from the group consisting of
      - a collection belt and belt guide rolls for operating the collection belt in a continuous loop and defining the machine direction
         and
      - a pair of counterrotating vacuum rolls forming a gap therebetween;
   - a first forming head for depositing first fibrous material from the first fibrous material supply onto the air permeable regions of the collection system;
   - a first compaction unit;
   - optionally a particulate material application system with a particulate material supply;
   - optionally a second forming head for depositing second fibrous material from the first or second fibrous material supply onto the air permeable regions of the collection system;
   - a first suction unit connected to the collection system to cooperate with the first forming head, optionally a second suction unit to cooperate with the second forming head, and optionally a further suction unit to cooperate with the particulate material application system;
   - a first transfer unit;
B- depositing in the deposition region of the first forming box a layer of first fibers from the first fibrous material supply and optionally particulate material from a first particulate material supply onto the air permeable regions of the collection system, whilst supporting the deposition by vacuum suction of the first vacuum suction system such that the blocked regions of the collection system are essentially free of fibrous material, thereby creating first fibrous web sub-sections;
C - transferring the first fibrous web sub-sections to and through the first compaction unit;
D - optionally transferring the first fibrous web sub-sections to a further particulate material application system and applying particulate material onto the first fibrous web sub-sections, thereby preferably leaving the blocked regions of the collector belt free of particulate material, preferably supported by suction of a further vacuum suction unit;
E - optionally transferring the first fibrous web sub-sections to and into the second forming box;
F - optionally depositing in the second forming box a layer of second fibers from the first or the second fibrous material supply and optionally particulate material from a second particulate material supply onto the first fibrous web sub-sections, whereby, supported by the second vacuum suction system,
   whereby the blocked regions (1518) of the collection system (1500) remain essentially free of fibrous or particulate material,
   thereby creating second fibrous web sections;
G - transferring the fibrous web sections comprising
   ∘ at least the first sub-sections, optionally comprising particulate material;
   ∘ optionally the second sub-sections, optionally comprising particulate material;
   ∘ optionally particulate material sandwiched between the first and the second web sub-sections
   to the transfer unit,
wherein the steps B to G are executed in the given order.

Steps B or F may be executed such that the fibrous material supply may be executed by individualizing fibers from a supply in a fiber board form by a hammer mill, thereby preferably orienting the axis of the hammer mill cross-directionally.

Preferably, in steps B or F, the fibrous material supply may be executed by enhancing the fiber flow by a venturi effect restriction in the fiber supply pipe.

Further, in steps B or F, the depositing of the fibers of the fibrous material may be supported by a fiber distribution homogenization unit comprising multiple fiber homogenization tools, preferably of the impeller type rotating around a vertical axis.

Optionally, in step A1 the step of providing a first fibrous material supply and optionally a second fibrous material supply may include the step of providing untreated cellulose fibers.

In another aspect, the present invention is a process for forming a laminate which comprises a series of web sections and further comprising the steps of
A2 - further providing,
   o a first envelope web from a first envelope web supply and a first envelope web combining roll, preferably a vacuum roll;
   o a second envelope web from as second envelope web supply and a second envelope web combining roll;
   ∘ optionally a first and a second glue applicator unit, adapted to apply glue to the first or the second envelope web, respectively;
   ∘ optionally a final bonding roll;
H - transferring the web sections towards and through the nip of a first and a second combining roll;
I - transferring the first envelope web from the first envelope supply unit to the first envelope combining roll, optionally applying thereby a glue from the first glue applicator unit;
K' - transferring the second envelope web to the second envelope web combining roll, thereby optionally applying a glue from the first glue applicator, and further into a nip between the second envelope web combining roll and the first envelope combining roll;
L' - enveloping the series of web sections in the gap between the combining roll in the first and second envelope web, thereby forming the laminate;
M - optionally transferring the laminate to a final bonding unit, preferably a melt-fusion bonding unit.

In yet another aspect, the present invention is a process of forming a laminate which further comprises the steps of
A2 - further providing,
   ∘ transfer roll;
   ∘ a second compaction unit comprising a nip formed between
      □ a first roll, preferably a patterned roll
      □ and a counteracting vacuum roll;
H - transferring the web sections to the transfer unit towards and through the nip of the second compaction unit;
I - transferring the first envelope web from the first envelope supply unit to the first envelope combining roll, optionally applying thereby a first glue from the first glue applicator unit;
K - transferring the compressed web sections to the first envelope combining roll(, thereby positioning it on the first envelope web;
L - transferring the second envelope web to the second envelope web combining roll, thereby optionally applying a second glue from the second glue applicator, and further into a nip between the second envelope web combining roll and the first envelope combining roll, thereby forming enveloped separated fibrous sections;
M - optionally transferring the enveloped separated sandwich web sections to a final bonding unit, preferably a melt-fusion bonding unit;
N - forming individualized articles by finishing up the enveloped separated sandwich web sections by
   ∘ fully separating consecutive sandwich web sections from each other and/or packaging these enveloped separated fibrous web sections;
   o packing it in roll-form with semi-separated separation lines between adjacent web sections,
wherein steps I to N are executed in the given order subsequent to step H.

In yet another aspect, the present invention is a process for forming a laminate comprising a series of web sections which further comprises the steps of
A2 - further providing,
   o a first envelope web from a first envelope web supply and optionally a first envelope web combining roll;
   o a second envelope web from a second web envelope web supply and a second envelope web combining roll;
   ∘ optionally a first and a second glue applicator unit, adapted to apply glue to the first or the second envelope web, respectively;
   ∘ optionally a final bonding roll;
   ∘ a finishing unit, preferably comprising a separation unit and a packaging unit;

   - I - transferring the first envelope web from the first envelope supply unit to the first glue applicator and selectively apply glue to glued portions of the first envelope web, separated by glue free portions, whereby the glued portions correspond in positioning to the air permeable regions of the collection system, and preferably exhibit x-y-directionally size of at least 5 mm less than the air permeable regions;
O - transferring the first envelope web towards the collection system at a speed corresponding to the speed of the collection system and positioning the glued portions over the air permeable regions, preferably such that a glue free periphery of at least 5 mm relative to the air permeable portions is formed;
B' - depositing in deposition region of the first forming box a layer of first fibers of the fibrous material from the first fibrous material supply and optionally particulate material from a first particulate material supply onto the first envelope web over the air permeable regions of the collection system, whilst supporting the deposition by vacuum suction of the first vacuum suction system such that the blocked regions of the collection system are essentially free of fibrous or particulate material, thereby creating first fibrous web sub-sections;
C - transferring the first fibrous web sub-sections to and through the first compaction unit;
D - optionally transferring the first fibrous web sub-sections to a further particulate material application system and applying particulate material onto the first fibrous web sub-sections, thereby preferably leaving the blocked regions of the collection system free of particulate material, preferably supported by suction of a further third vacuum suction unit;
E - optionally transferring the first fibrous web sub-sections to and into the second forming box;
F - optionally depositing in the second forming box a layer of second fibers of the fibrous material from the first or the second fibrous material supply and optionally particulate material from a second particulate material supply onto the first fibrous web sub-sections, whereby, supported by the second vacuum suction unit,
   o the blocked regions of the collection system remain essentially free of fibrous and particulate material,
   thereby creating second fibrous web sections;
G' - transferring the fibrous web sections comprising
   ∘ at least the first sub-sections, optionally comprising particulate material;
   ∘ optionally the second sub-sections, optionally comprising particulate material;
   ∘ optionally particulate material sandwiched between the first and the second web sub-sections
   on the first envelope web to the second envelope web combining roll;
K'- transferring the second envelope web to the second envelope web combining roll, thereby optionally applying a second glue from the second glue applicator;
L' - combining the series of web sections on the first envelope web with the second envelope web, thereby forming the laminate;
M - optionally transferring the laminate to a final bonding unit, preferably a melt-fusion bonding unit.

In an even further aspect of the present invention, the process of forming a laminate may further comprise the steps of
A2 - further providing
   ∘ a transfer roll;
   ∘ a second compaction unit comprising a nip formed between
      □ a first roll, preferably a patterned roll
      □ and a counteracting vacuum roll;
H - transferring the web sections on the first envelope web to the transfer unit towards and through the nip of the second compaction unit, thereby forming a series of embossed web sections;
L - transferring the second envelope web to the second envelope web combining roll, thereby optionally applying the second glue from the second glue applicator (2420), and combining it with the series of embossed web sections by the combining roll, thereby forming enveloped separated fibrous sections;
M - optionally transferring the enveloped separated sandwich web sections to a final bonding unit, preferably a melt-fusion bonding unit;

In yet a further aspect, the present invention is the converting of such a laminate into a series of articles, by further executing the steps of
N1 - forming individualized articles by finishing up the enveloped separated sandwich web sections by fully separating consecutive sandwich web sections from each other and/or packaging these enveloped separated fibrous structures,
   or
N2 packing the laminate in roll-form with semi-separated separation lines between adjacent articles.

### Brief description of the Figures

Fig. 1 shows an equipment for operating the present invention of forming a series of separated fibrous web sections.
Fig. 2 A and B show further details of the equipment for operating the present invention of forming a series of separated fibrous web sections.
Fig. 3 depicts a further execution of an equipment for operating the present invention of forming a series of separated fibrous web sections.
Fig. 4 depicts an equipment for forming a laminate according to another aspect of the present invention comprising separated fibrous web sections.
Fig. 5 A to D depicts a further equipment for forming a laminate according to another aspect of the present invention comprising separated fibrous web sections.
Fig. 6 depicts a variant of the present invention for embossing a series of separated web sections.
Fig. 7 depicts a further variant of forming a series of separated web sections.

The figures are schematic only, and not to scale. Same numerals refer to same or equivalent features or elements, single (') or multiple (", ‴, ...) apostrophes indicate duplicate features, such a left and right or front and back, etc. Omitting features in one figure does not imply that these cannot be combined with features of other figures.

### Detailed description

The present invention relates to a process for forming a series of separated fibrous web sections comprising fibrous material, especially short fibers, such as cellulosic fibers.

Wood pulp fibers are a preferred starting material as may be formed by a variety of pulping processes, such as kraft pulp, sulfite pulp, thermomechanical pulp, and the like. Further, the wood fibers may be any high-average fiber length wood pulp, low-average fiber length wood pulp, or mixtures of the same. One example of suitable high-average length wood pulp fibers includes softwood fibers such as, but not limited to, northern softwood, southern softwood, redwood, red cedar, hemlock, pine (e.g., southern pines), spruce (e.g., black spruce), combinations thereof, and the like. One example of suitable low-average length wood pulp fibers includes hardwood fibers, such as, but not limited to, eucalyptus, maple, birch, aspen, and the like. Such fibers may exhibit an average fiber length, as may be determined by using a Kajaani Fiber Analyzer, of less than about 2.5 mm, or less than about 1.5 mm or less than about 1.0 mm The fibers may be treated so as to allow optimization of processing and or absorbency properties, The term "treated" as used herein is understood to include any means of introducing the additive to the fiber and/or fibrous matrix, but not limited to, such as coating, spraying, printing, chemical modifications, wet-end additions applications to the fibers as well as blending untreated fibers with treated fibers.

Moreover, if desired, secondary fibers obtained from recycled materials may be used, such as fiber pulp from sources such as, for example, newsprint, reclaimed paperboard, and office waste, or recycled diapers, be it from factory scrap or be it post-consumer recycling.

The fibrous material may be delivered as fibrous board or in fibrous sheet form. Also bales of fibrous material may be delivered as may be opened prior to be used in the process according to the present invention. Further, "roughly graded material" may be employed, wherein fibers are present as clusters of several hundred up to several thousand fibers in the roughly graded material. It is an object of the present invention to dry lay, or air lay, such fibrous material to form fibrous webs. The fibrous webs may further comprise particulate material. In particular for absorbent articles, such particulate material may be superabsorbent polymer (SAP) particles, as well-known in the art. Such particulate material may be homogeneously intermixed into fibrous web, i.e., positioned in the interstitial pores of a fibrous web or of a portion or layer of such a fibrous web. Such particulate material may also be positioned between sub-layers of a fibrous web, thereby optionally partially penetrating into one or more of the layers.

Within the present context, the term "fibrous web" encompassed fibrous webs without and with particulate material, as may further be converted into articles comprising such fibrous webs or sections thereof.

Such articles comprise further materials, typically web materials for stabilizing and/or containing the fibrous webs. Conventional air-laying processes are often operated in an "off-line" modus, whereby jumbo reels of the fibrous structure are produced on machines with a large width. Such wide roll may be cut to a lower width and rewound for being transferred to a converter, where the fibrous materials are cut to the appropriate size for being introduced into articles, often absorbent articles, such as diapers, wipes, or pads like bed-pads, or the like.

Whilst such processes allow efficient production of the fibrous webs, rewinding and transport imply process complexity and cost. When avoiding this by producing airlaid webs in-line on the converting process, conventional air-laying processes provide continuous webs that imply potential for contamination when being cut to the right length, and further require cut and space operation to feed into the finished articles.

In first aspects, the present invention is an air-laying process of producing a series of separated fibrous web sections for being processed in further processing steps for forming articles comprising fibrous structures.

In further aspects, the present invention is an equipment for and a process for converting airlaid fibrous web sections into a laminate, whereby fibrous web sections are separated by a region which is free of the airlaid fibrous structure, but essentially only comprises envelope webs, such as a backsheet, and a cover web such as a topsheet. The fibrous web sections may be a single layer of a fibrous web, optionally comprising particulate material intermixed or positioned onto a surface thereof. In a particular execution, the airlaid fibrous web sections are made up of two airlaid fibrous web sections, preferably differing in density, and further optionally comprising particulate material in either of the fibrous webs or therebetween, such as superabsorbent polymers (SAP) in particulate form, including fibrous form.

In a particular execution and in contrast to conventional structures, the ones as made according to the present invention may be free of a supporting substrate, such as typically tissues, that not only enable a conventional continuous production but also prevent SAP particles to give a rough feel for the backsheet side and may even damage backsheets. Similarly, conventional structures also comprise a user-oriented preformed cover web, typically a paper tissue, for preventing SAP particles to penetrate through to user's skin. Thus, such backsheet side and topsheet side tissues may be readily omitted when sandwiching or laminating the fibrous web sections between envelope webs such as topsheets and backsheets. Such laminates comprising separated web sections sandwiched between envelope webs may then be readily transformed into finished articles, such as - without limitation - bed pads.

In another aspect the present invention is an equipment to produce a series of such separated airlaid fibrous web sections. It is an important element of such an equipment that the collection surface 1505 on which the fibrous material and optionally particulate material are forming the fibrous web exhibit regions of varying air permeabilities, preferably regions with and regions without or at least significantly reduced air permeability. In particular executions, these sections of fibrous webs may be embossed, as may create bonding between fibers, and sandwiched between envelope webs such as topsheet and backsheet, to further form articles, which can be readily finished by separation, folding and packaging operations.

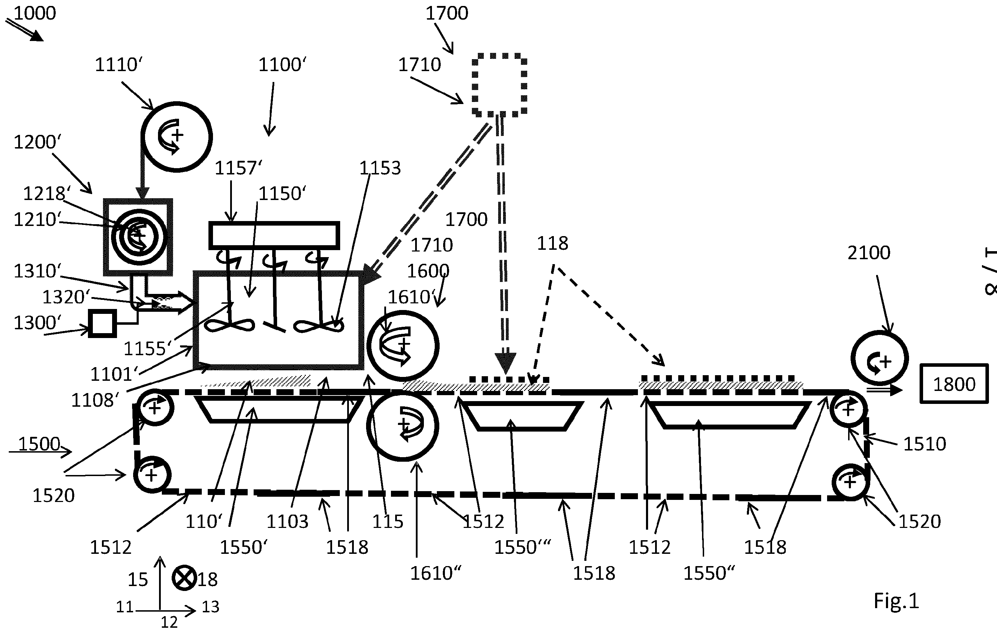

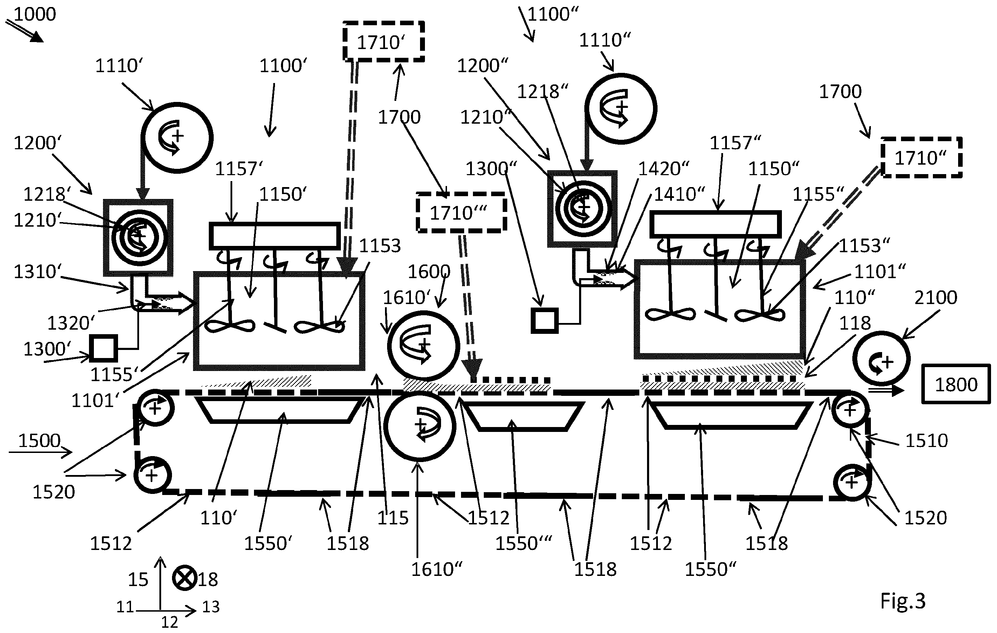

In order to further explain the present invention, reference is made to Fig. 1 for a single forming head and Fig. 3 for an option with a dual forming head set-up, which however, should not be seen to limit the present invention.

An equipment 1000 for forming a series of fibrous airlaid webs 110 comprises at least one web forming head 1100' and may optionally comprise a further web forming head 1100" (see Fig. 3). These may be conventional air-laying heads, as known from the prior art as discussed in the above. As indicated in the Fig 3, the web forming heads 1100', 1100" may be essentially identical in design, and even may be operated at identical process conditions, but these could also have differing designs and process settings.

The equipment exhibits an overall process or machine (x-) direction 12, a width or cross-machine direction (y-) 18, and a height (z-) direction 15 perpendicular to both and aligned with gravity.

Generally, the webs or materials are moved from an upstream portion 11 of the process or machine downstream 13, at least partially along the machine direction 12.

As exemplarily shown in Fig. 1 and 3, the fibrous material is delivered from a fibrous material supply 1110', 1110" towards a disintegration unit 1200', 1200"

In a particular execution, the fibrous material supply 1110', 1110" is in the form of a compressed fiber board roll, and the disintegration unit is a hammer mill 1210, and preferably the rotating axis of the hammer mill is along the width or y-direction of the overall equipment. However, other fibrous material supply options may be employed, such as using fiber bales, from which fibers may be individualized by picking teeth.

The fibers of the fibrous material are transferred, preferably by pneumatic transfer in piping 1310, preferably further aided by additional fiber supply air 1300 and even more preferably by a venturi effect restriction 1320.

The forming heads 1100, 1100" may comprise a forming box 1101', 1101" with one or more inlet openings 1102', 1102", respectively, for receiving the individualized fibers and an outlet 1108', 1108" through which the individualized fibers are deposited. The forming heads preferably comprise a fiber distribution homogenization unit 1150', 1150" for homogenizing the x-y-directional distribution of the fibers.

In a preferred execution the fiber distribution homogenization units 1150', 1150" comprise a multiplicity of fans 1153', 1153" rotating by means of drives 1157', 1157" around vertical axes 1155', 1155". As indicated in the cross-sectional view of Fig.1 and 3, each three such fans 1153 are shown in each of the forming heads 1100', 1100", but - as indicated in the background section - there could be an array of fans, with two, three, four or even more arranged in the cross-directional rows and two, or four, or six, or eight or ten or even more arranged in machine-directional columns. Optionally, the fans in adjacent rows may be cross-directionally off-set, or the rows and columns are in an angled position relative to the machine direction. The drives 1157', 1157" for the fans may be a single one connected to the fans by belts or gear wheels, or multiple drives (not shown). Optionally, particulate material may be inserted in one or both from a particulate material application system 1700', 1700" from particulate material supply 1710', 1710" into the forming box 1101', 1101" for being intermixed with the fibers.

The individualized and x-y-directionally evenly distributed fibers and particulate material, if present, are deposited though the forming box outlets 1108', 1108" onto the collection system surface 1505 of the collection system 1500, here shown as a collection belt 1510, further guided by first (1550') and second (1550") vacuum suction systems, respectively. It is an important aspect of the present invention that the collection surface 1505 of the collection system 1500, such as the collection belt 1510 comprises alternating regions with high air permeability 1512 and blocked regions with low, or preferably no air permeability 1518. Thus, the machine-directional distribution of the fibers is modified, as the air-entrained fibers are directed within the forming boxes 1101', 1101" with the air stream towards the high permeability regions 1512, there forming fibrous web sections 110', 110" with machine directionally increasing thickness but with fiber free regions 115 between neighbouring web sections 110.

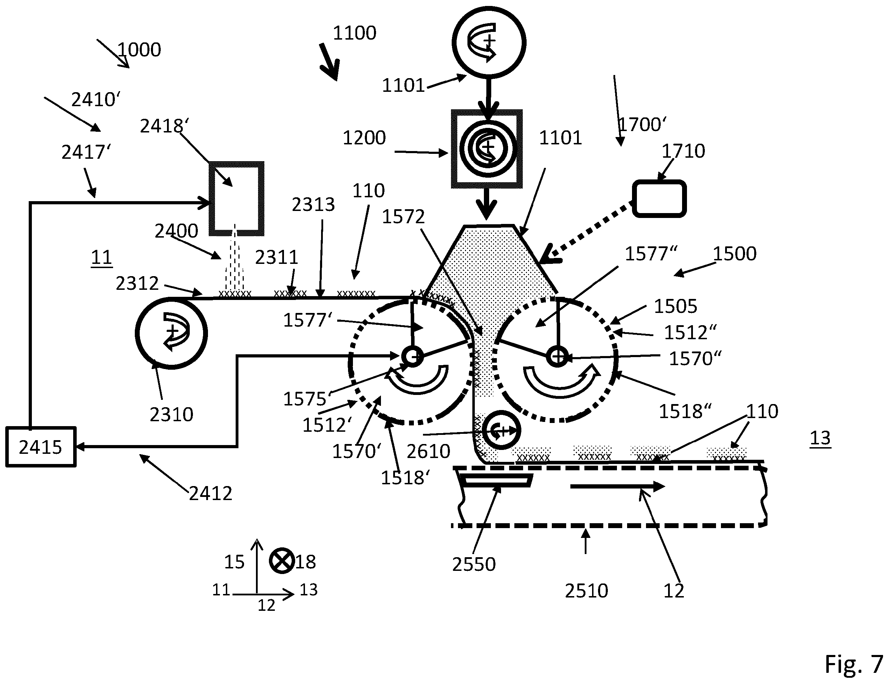

As will be discussed hereinbelow in more details, Fig. 7 depicts a variant of a collection system 1500, comprising two counterrotating drums 1570', 1570" with vacuum zones 1577', 1577", wherein the collection system surface 1505 comprises air permeable regions 1512 and air impermeable blocked regions 1518.

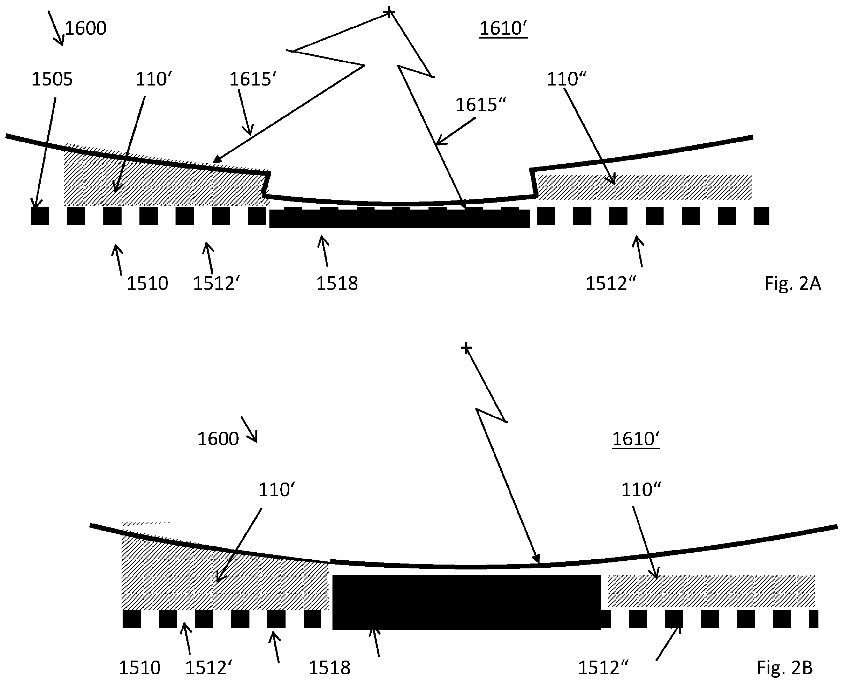

Referring also to Fig. 2A and 2B, the low permeability blocked regions 1518 may be manufactured by a multitude of approaches, such as by applying a blocking onto the collection surface 1505 of the collection system surface such as fixing a strip of solid material (Fig. 2A), or applying a blocking material such as glue or silicones into the pores of the foraminous belt 1510 (see Fig. 2A) or the vacuum drum shell. Also, when the collection belt is manufactured of a thermoplastic material, the low permeability regions may be achieved by compressing the material in these regions, thereby forming air impermeable film like structures.

However, when the collection system comprises a collection belt 1510, this runs over various guide rolls and through compression nips (as will be discussed herein below), and it is a particularly preferred execution that the collection 1510 belt exhibits an essentially equal thickness in the permeable (1512) and less or non-permeable blocked (1518) regions. Such impermeable regions may be created by impregnating the collection belt in the relevant regions with a curable resin, as well known in the art, see e.g., WO2020/256714 (P&G), to which express reference is made for the description of formation of the airflow blocking regions 1518.

The equipment 1000 further comprises a compaction unit 1600 positioned after the first forming head (1100') and the second forming head 1100", if present, and adapted to compress the web sections 110', indicated by to counterrotating rolls 1610, 1620, both of which may exhibit a smooth surface thus creating a flat surface of the web sections. This aspect is of particular importance, when the web sections 110 are compacted after the forming head 1100 by a first compaction system 1600. As indicated in Fig. 2A exemplary for a collections system 1500 with a collection belt 1510, the upper compression roll 1610' in contact with the web may exhibit a first diameter 1615' corresponding to and operated in phase with permeable regions 1512 of the foraminous belt 1510, and a second, larger diameter 1615" corresponding to and operated phased with the blocked regions 1518 of the foraminous belt 1510. The difference in diameter can be adjusted to the desired degree of compression. As indicated in Fig. 2B, the compression roll may have a constant diameter 1615, which may counteract with raised blocking regions 1518 of the foraminous belt 1510, whereby the protrusions of the blocked regions 1518 determines the compression.

In addition or alternative to adding particulate material into the forming boxes 1101' and/or 1101", a further particulate material application system 1700‴ comprising a further particulate material supply 1710'", may be positioned preferably between the compaction unit 1600 and the second forming head 1100", adapted to apply particulate material 118, as may be superabsorbent polymers, such as SAP particles, onto the first formed web sections 110'. There is no particular limitation to the selection of such a particular material applications system. Exemplary executions include continuous or discontinuous rotary or slot applicators, optionally supported by doctor blades. Other systems include powder spray systems. For particular applications, it may be preferred to apply the particles in a pattern, such as may by well-known particle printing processes. It is highly preferred that the regions between the web sections also remain free of particulate material, e.g., via the patterned application tools. When the particles are applied in a continuous manner, the airflow blocking structures 1518 may also prevent the particles from depositing there. If this is not sufficient, particle removal tools (not shown) may be employed, such as blowing air or brushing particles away.

The equipment further comprises a web section removal unit adapted to receive the fibrous web sections 110 and transferring these by means of a pick-up and transfer unit 2100, preferably a vacuum drum, to further web processing units 1800.

A particular execution of a web processing unit is an enveloping unit for laminating or sandwiching the separated fibrous web sections 110 between envelope webs, such as a first envelope web 2312 as a cover material or a topsheet, as may be a liquid and air permeable web, such as a nonwoven, and a second envelope web 2322 as a carrier material or backsheet, such as a liquid impermeable web, e.g., a film material.

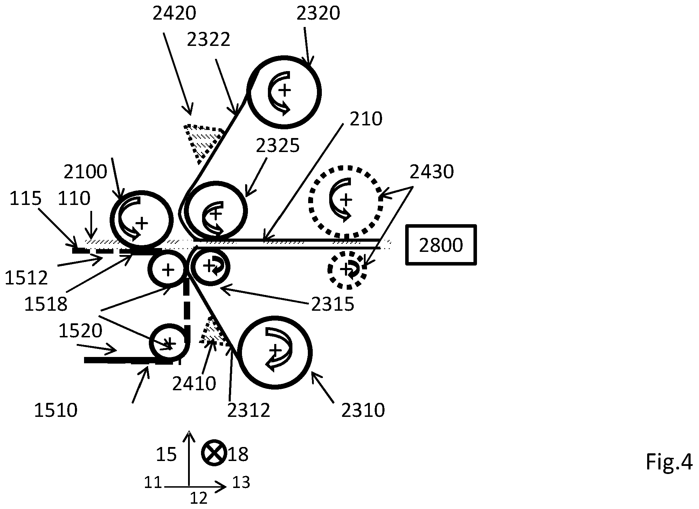

Such a further processing unit for laminating executing further processing steps is indicated in Fig.4. To this end, the web sections 110, separated by fiber free regions 115, are transferred, e.g., by a transfer roll 2100, into a gap formed by combining rolls 2315 and 2325, to which envelope webs 2312 and 2322, respectively, are suppled from envelope supplies 2310, 2320, respectively, both optionally with a glue or adhesive applied from glue applicators 2410 and 2420, respectively. At least one of the glue applicators may apply glue to areas surrounding the web sections 110, such that the laminate may be fixed, optionally by a final bonding unit 2430. Additionally or alternatively, one or both of the glue application(s) may be omitted, and the bonding may be achieved in the final bonding unit 2430, e.g., by thermal, pressure, or ultrasonic bonding,

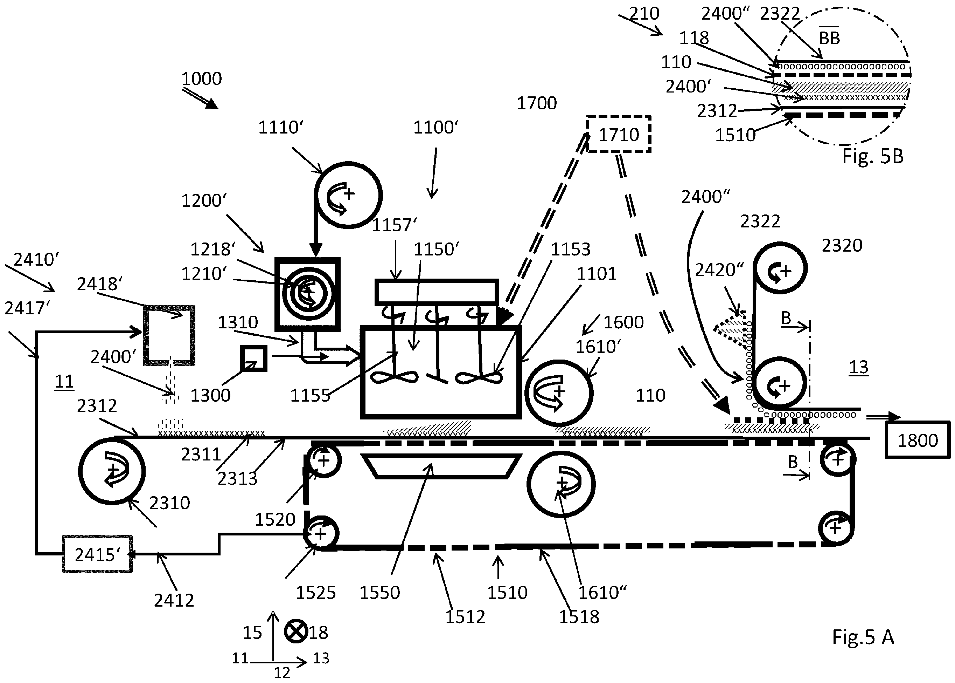

An alternative approach to the laminating is indicated in Fig. 5 A to D, and Fig. 7 with unwinding a first envelope web 2312 from a first envelope web supply 2310 and feeding this via the collection surface 1505 of a collection system 1500 to the first forming box 1101'. As shown in Fig. 5A for a collection belt 1510 and Fig. 7 for a collection system with two counterrotating drums 1570' and 1570", glue 2400 can be applied to the first envelope web onto the surface onto which the fiber will be deposited. However, in order not to contaminate the further downstream process steps like compacting, the glue is applied in a pattern so as to create glued (2311) and un-glued (2313) portions of the web. The first ones correspond in position and size to the intended size of the fibrous web sections, which in turn correspond to the size of the air permeable regions 1512 of the surface of the collection system - except that the glued portions are somewhat smaller so as to create a glue free perimeter 2405 around the web sections. Over and beyond the glue free sections between neighbouring web sections, the glue free perimeter 2405 is preferably more than about 5 mm, or more than about 10 mm, but less than about 20 mm relative to the size of the web sections 110 or the air permeable section 1512, respectively. It is important, that the phased glue is applied synchronously to the web section size and hence to the size of the air permeable (1512) and impermeable (1518) regions of the foraminous belt 1510, as well as of the production speed. This is achieved by an glue application control system 2415, which - in addition to be programmed to the appropriate article size - automatically adjusts glue application to the foraminous belt speed as transmitted via speed signal 2412. The glue application system with application nozzle 2418 which receives the glue nozzle application signal 2417 may be a conventional high frequency, drip free hot melt applicator system, such as available from Nordson Corp., OH, USA, or ITW Dynatec, TN, USA.

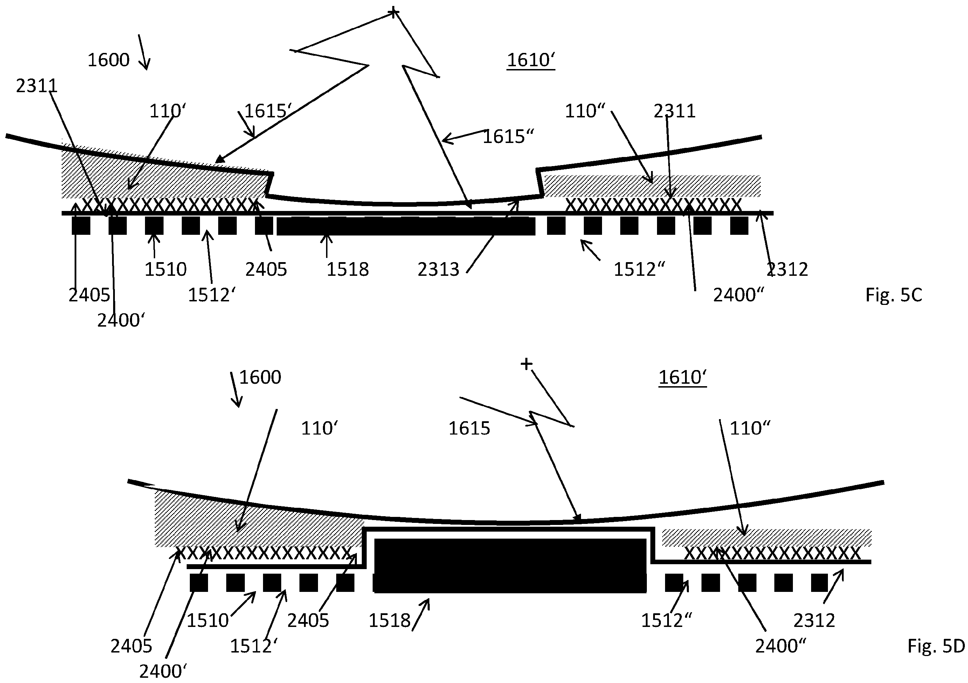

Fig. 5C and D depict the optional execution of raised or flat non-permeable regions 1518 of the collection surface 1505, e.g., for a collection belt 1510, showing the first envelope web 2312, the glued regions 2311 and the glue free regions 2313 including the glue free perimeter 2405 circumscribing the glued regions 2311 underneath the deposited web sections 110.

As shown in Fig. 5A, particulate material may be applied in an optional particulate material application system 1700 and the web sections 110 may then be transferred the laminating section, where the second envelope web 2322 can be applied as described in the above by being unwound from a second envelope web supply 2320, optionally glue can be applied by second glue application system 2420 and transferred to further downstream processing 2800.

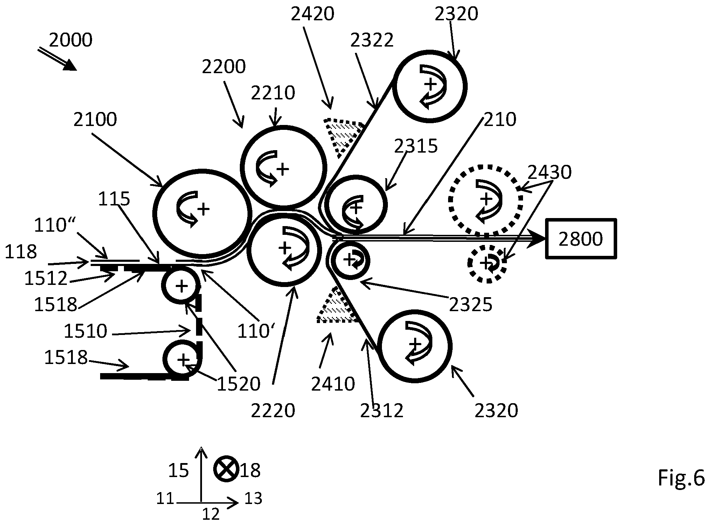

In yet a further variant, the web sections 110 may be treated by a further a compaction system 2200 for further compacting the web sections 110, be these on a first envelope web 2312 or not. For the latter options, such a compaction, as may be executed in a multitude of known alternatives, is explained by referring to Fig. 6, such as by feeding web sections 110 into a nip between an embossing roll 2210, preferably a patterned embossing roll, and a counteracting vacuum roll 2220, preferably with a smooth, perforated surface. After the embossing, a first envelope web 2312, as may be referred to as a topsheet, may be unwound from a first envelope supply 2310 and fed towards a first envelope web combining roll 2315, preferably also a vacuum roll. As the width of this web material is wider and the pitch of this material is longer than the separated web sections, a perimeter free of the fibrous and particulate material is formed. Whilst the web sections and the first enveloping web material are hold on the first envelope web combining roll 2315, a second enveloping web is provided from a second enveloping web supply 2320 and combined with the first enveloping web and the web sections positioned thereon, thus forming a series of individualized fibrous web sections 110, separated by fiber and particle free regions 115. The structure may be held together by conventional means, such as by glue as may be applied to the first (2312) and/or second (2322) envelope web, and/or by melt-fusion bonding applied in a further processing step 2430, these options being indicated in Fig. 6 with dotted lines. A particular execution for the embossing equipment and its operation is described in more detail in co-pending application EP25169008 (CoAx), to which express reference is made with regard to the embossing of fibrous web sections.

The laminated fibrous web sections may then be further transferred to further processing units 2800, as may be a finishing and packaging unit, optionally with a separation unit to separate the individualized structures in the fiber and particle free regions 115.

Referring again to Fig 6, a further variant for executing the present invention is depicted. As shown with the option of depositing the fibrous web sections 110 onto a first envelope web 2312 with pre-applied phased glued regions 2311, this web is fed towards a set of counterrotating drums 1570' and 1570", each with a collection surface 1505 comprising air permeable regions 1512 and air impermeable regions 1518. The drums comprise vacuum sections 1577' and 1577", corresponding to the web deposition region 1103 of the belt systems as described hereinabove. Also, in analogy to the above, the phased glue is applied synchronously to the web section size and hence to the size of the air permeable (1512) and impermeable (1518) regions of the drums 1570' and 1570", as well as of the production speed. The individualized fibers are supplied from a fibrous material supply 1110 to a forming box 1101, optionally intermixed with particulate material as may be added thereto from a particulate material supply 1710 and - aided by the vacuum suction - deposited onto the deposition regions 1103' as being covered by air permeable envelope web 2312 with glued (2311) and unglued (2313) regions, respectively. The deposited fibers are fed by gravity and rotation of the drums 1570 into the gap 1579 between the drums towards a further web transfer unit, here shown as a foraminous belt 2510 with a further vacuum system 2550, where further compaction and/or combining with a second envelope web may be execute (not shown in Fig 6). By holding the transfer distances short, the applied glue will suffice to maintain the web sections intact.

Thus, the process for forming a series of fibrous web sections 110 according to the present invention can be executed on an equipment as described in the above by the following steps:
- disintegrating the fibers of the fibrous material in a disintegration unit 1200' of a first forming head 1100'. In a preferred execution, the disintegration unit 1200' is arranged such that the individualized fibers are moved along the machine direction towards the forming head 1100' by positioning the rotating axis of the disintegration unit 1200' cross-directionally.
- feeding the individualized fibers pneumatically via connection pipe 1310 and preferably with additional air of the air supply 1300' and more preferably via a venturi effect flow path restriction 1320 towards an opening 1102' of the first forming head 1100'.
- homogenizing the x-y-directional distribution of the fibers in the first forming head 1100' by the first fiber distribution homogenization system 1150'.
- forming first fibrous sub-sections 110' by depositing the fibers and optionally intermixed particular material from a first particular material supply 1710' through the forming box outlet 1108' onto the air permeable regions 1512 of the collection system 1500, such as a collection belt 1510 or a pair of collection drums 1570', 1570", thereby supported by the first vacuum suction system 1550' and thus forming a series of first fibrous web sub-sections 110', separated by essentially fiber free regions 115, thereby executing the separation such that a cross-directionally extending region extending over the full width of the fibrous sections.
- transferring the series of first fibrous web sub-sections 110' towards a first compaction unit 1600 and compressing the first fibrous web sub-sections 110'.
- optionally transferring the compressed first fibrous web sections 110' to the further particulate material application system 1700‴ and applying the particulate material 118 onto the first fibrous web sub-sections 110'. Due to the vacuum suction unit 1550"', the regions being essentially free of fibers remain also essentially free of particulate material.
- optionally transferring the first fibrous web sub-sections 110', optionally with the particulate material 118 towards a second forming head 1100", and applying second fibrous web sub-sections 110" in analogy to forming the first fibrous web sub-sections110' as described in the above. The material selections and the process settings may be, but do not need to be, the same as for the first forming head 1100'.
- transferring the series of separated web sections 110, comprising the first (110') and optionally the second (110") web sub-sections, optionally each comprising particulate material, and optionally the particulate material 118 sandwiched therebetween, to a pick-up and transfer system 2100 and removing the web sections 110 from the transfer belt 1510, preferably by applying vacuum suction in a transfer roll 2100 with a foraminous surface. The series of web sections may then further be transferred to further web handling units 1800.

In another aspect, the process of forming a laminate 210 comprising a series of fibrous web sections 110 according to the present invention comprises the steps of
- providing a series of web sections 110 as described in the above;
- providing further,
   o a first envelope web 2312 from a first envelope web supply 2310 and a first envelope web combining roll 2315, preferably a vacuum roll;
   o a second envelope web 2322 from as second envelope web supply 2320 and a second envelope web combining roll 2325;
   ∘ optionally a first 2410 and a second 2420 glue applicator unit, adapted to apply glue to the first 2312 or the second 2322 envelope web, respectively;
   ∘ optionally a final bonding roll 2430;
- transferring the web sections 110 towards and through the nip of a first 2315 and a second 2325 combining roll;
- transferring the first envelope web from the first envelope supply unit 2310 to the first envelope combining roll 2315, optionally applying thereby an glue 2400' from the first glue applicator 2410;
- transferring the second envelope web to the second envelope web combining roll 2325, thereby optionally applying a glue 2400" from the first glue applicator 2420, and further into a nip between the second envelope web combining roll 2325 and the first envelope combining roll 2315;
- enveloping the series of web sections 110 in the gap between the combining roll 2315, 2325 in the first 2312 and second 2322 envelope web, thereby forming the laminate 210;
optionally transferring the laminate to a final bonding unit 2430, preferably a melt-fusion bonding unit.

Optionally, the series of web sections 110 may be submitted to a further compression step prior to being enveloped, thereby comprising the steps of
- providing a laminate 210 of a series of fibrous web sections 110
- providing further
   o transfer roll 2100;
   o a second compaction unit 2200 comprising a nip formed between
      □ a first roll, preferably a patterned roll 2210
      □ and a counteracting vacuum roll 2220;
- transferring the web sections 110 to the transfer unit 2100 towards and through the nip of the second compaction unit 2200 prior to transferring it into the gap between the first 2315 and second 2325 combining roll:

Instead of laminating the series of web sections 110 concurrently between the first 2312 and second 2322 envelope web, the first envelope web 2312 may already be fed into the first forming box and the fibers be deposited thereon. Such a process variant comprises the steps of
- transferring the first envelope web from the first envelope supply unit 2310 to the first glue applicator 2410 and selectively apply glue 2400 to glued portions 2311 of the first envelope web 2312, separated by glue free portions 2313,
   whereby the glued portions 2311 correspond in positioning to the air permeable regions 1512 of the collection system 1500, and preferably exhibit x-y-directionally size of at least 5 mm less than the air permeable regions 1512
- transferring the first envelope web 2312 towards the collection system 1500 at a speed corresponding to the speed of the collection system 1500 and positioning the glued portions 1311 over the air permeable regions 1512, preferably such that a glue free periphery of at least 5 mm relative to the air permeable portions 1512 is formed;
- forming separated web sections 110 on said first envelope web, as described in the above;
- transferring the fibrous web sections 110 on the first envelope web 2312 to the second envelope web combining roll 2325;
- transferring the second envelope web to the second envelope web combining roll 2325, thereby optionally applying an glue 2400" from the first glue applicator 2420;
- combining the series of web sections 110 on the first envelope web 2312 with the second envelope web 2322, thereby forming the laminate 210.

Also for this process variant, an embossing step for the web sections 110 may be performed prior to be laminated with the second envelope web 2322, by comprising the processing steps of
- providing series of fibrous web sections 110 positioned on a first envelope web 2312;
- providing further
   o transfer roll 2100;
   o a second compaction unit 2200 comprising a nip formed between
      □ a first roll, preferably a patterned roll 2210
      □ and a counteracting vacuum roll 2220;
- transferring the first envelope web 2312 with the web sections 110 positioned thereon to the transfer unit 2100 towards and through the nip of the second compaction unit 2200 prior to transferring it into the gap between the first (2315) and second (2325) combining roll.

In yet a further aspect, the present invention is particularly suitable for being integrated into a converting process for forming articles, such as bed-pads, or wipes, or absorbent articles, as it obviates the need of processing the "jumbo roll" webs of conventional air-laying equipment and transporting the pre-formed webs to the article forming converter lines.

Thus, it is a particular effect of the present invention, that separated air-laid web sections 110 may be directly fed into a further equipment units and processing steps for manufacturing articles comprising such webs sections, such as without limitation bed-pads, or wipes, or feminine hygiene pads, or other absorbent articles like diapers or adult incontinence articles.

To this end, the individualized articles may be formed by finishing up the laminate 210, which comprises the enveloped separated sandwich web sections 110 by fully separating consecutive sandwich web sections from each other and/or packaging these enveloped separated fibrous structures. Alternatively, the laminate 210 may be packed in roll-form with semi-separated separation lines between adjacent enveloped separated sandwich web sections 110.

Particular articles that may be readily manufactured according to the present invention are bed-pads, that may exhibit
- an overall article length of more than about 300 mm, or more than about 600 mm, or more than about 800 mm, but typically less than about 1500 mm.
- an overall article width of more than about 100 mm, or more than about 250 mm, or more than about 400 mm, but typically less than about 1000 mm.
- a basis weight of the sandwich structure of more than about 30 g/m² or more than about 50 g/m² or more than about 80 g/m, but typically less than about 300 g/m².
- a weight ratio of the particulate material relative to the total fiber weight in the sandwich structure of more than about 5 w-%, or more than about 10 w-%, or more than about 20 w-%, or more than about 30 w-%, or more than about 50 w-%. Or even more than about 60 w-%

In a preferred design, the article consists essentially of the sandwich structure, a topsheet, a backsheet and glues, whereby the latter may be absent in case of thermo-fusion bonding, such as by heat-, pressure-, or ultrasonic bonding. A particular example for such an article may be a bed-pad, exhibiting
- an overall article length of 900 mm and width of 600 mm;
- an absorbent structure length of 830 mm and a width of 530 mm;
- an overall weight of the absorbent structure of about 50 g with about 17.5 g of pulp as a first (lower or backsheet side) layer, SAP weight of about 15 g, and about 17.5 g of pulp as a second (upper, topsheet side) layer.

It should be noted that the current invention is very flexible to adjusting the width of the formed webs independently of the type of fibrous material supply. Thus, the width of the fibrous material supply may be less than about 90 %, or less than about 70 %, or less than about 50 % or less than about 30 % of the width of the resulting web 100. In an exemplary execution, the fibrous material was supplied from a fiber board roll of a width of 500 mm, but can be converted into a web having a width of 830 mm - a suitable size for forming, e.g., bed-pads.

## Claims

**1.** An equipment for forming a series of separated fibrous sections (119), exhibiting a machine (x-) direction (12), a cross (y-) direction (18) and a height (z-)direction (15) and comprising
- a first fibrous material supply (1110') and optionally a second fibrous material supply (1110");
- a collection system (1500) comprising a collection surface (1505) comprising a plurality of air permeable regions (1512) and blocked regions (1518), which are essentially non-permeable to air, selected from the group consisting of
- a collection belt (1510) comprising belt guide rolls (1520) for operating said collection belt (1510) in a continuous loop and defining said machine direction (12),
and
- a pair of counterrotating vacuum drums (1570', 1570") forming a gap (1572) therebetween;
- a first forming head (1100') comprising a first forming box (1101') for depositing first fibrous material from said first fibrous material supply (1110') in a first deposition region (1103') onto said air permeable regions (1512) of said collection system (1500);
- a first compaction unit (1600);
- optionally a particulate material application system (1700) with a particulate material supply (1710);
- optionally a second forming head (1100") for depositing second fibrous material from said second fibrous material supply (1110") onto said air permeable regions (1512) of said collection system (1500);
- a first suction unit (1550') connected to said collection system (1500) to cooperate with said first forming head (1100'), optionally a second suction unit (1550") to cooperate with said second forming head (1100"), and an optionally further suction unit (1550"') to cooperate with said further particulate material supply (1710‴);
- a first transfer unit (2100),
**characterized in that** said equipment further comprises
- a collection system (1500) comprising a collection surface (1505) comprising a plurality or air permeable regions (1512) and blocked regions (1518), which are essentially non-permeable to air, selected from the group consisting of
- a collection belt (1510) comprising belt guide rolls (1520) for operating said collection belt (1510) in a continuous loop and defining said machine direction (12);
- a pair of counterrotating vacuum drums (1570', 1570") forming a gap (1572) therebetween.

**2.** An equipment for forming a series of separated fibrous sections (1100) according to claim 1, whereby said collection belt (1510) of said collection system (1500) exhibits an essentially constant thickness across its air permeable regions (1512) and blocked regions (1518).

**3.** A process for forming a series of separated fibrous sections (110), comprising the steps of
A1 - providing web forming equipment exhibiting a machine (x-)direction (12), a cross (y-) direction (18) and a height (z-)direction (15) and comprising:
- a first fibrous material supply (1110') and optionally a second fibrous material supply (1110");
- a collection system (1500), comprising a collection surface comprising a plurality of air permeable regions (1512) and blocked regions (1518) which are preferably non-permeable to air, selected from the group consisting of
- a collection belt (1510) comprising and belt guide rolls (1520) for operating said collection belt (1510) in a continuous loop and defining the machine direction (12),
and
- a pair of counterrotating vacuum rolls (1570', 1570") forming a gap (1572) therebetween;
- a first forming head (1100') for depositing first fibrous material from said first fibrous material supply (1110') onto said air permeable regions (1512) of said collection system (1500);
- a first compaction unit (1600);
- optionally a particulate material application system (1700) with a particulate material supply (1710);
- optionally a second forming head (1100") for depositing second fibrous material from said first (1110') or second fibrous material supply (1110") onto said air permeable regions (1512) of said collection system (1500);
- a first suction unit (1550') connected to said collection system (1500) to cooperate with said first forming head (1100'), optionally a second suction unit (1550") to cooperate with said second forming head (1100"), and optionally a further suction unit (1550"') to cooperate with said particulate material application system (1700);
- a first transfer unit (2100);
B- depositing in said deposition region (1103) of said first forming box (1100') a layer of first fibrous materials from said first fibrous material supply (1110') and optionally particulate material from said first particulate material supply (1710') onto the air permeable regions (1512) of said collection system (1500), whilst supporting the deposition by vacuum suction of said first vacuum suction system (1500') such that said blocked regions (1518) of said collection system (1550) are essentially free of fibrous material, thereby creating first fibrous web sub-sections (110');
C - transferring said first fibrous web sub-sections (110') to and through said first compaction unit (1600);
D - optionally transferring said first fibrous web sub-sections (110') to a further particulate material application system (1700‴) and applying particulate material onto the first fibrous web sub-sections (110'), thereby preferably leaving said blocked regions (1518) of said collector belt (1510) free of particulate material, preferably supported by suction of a further vacuum suction unit (1550‴);
E - optionally transferring said first fibrous web sub-sections (110') to and into said second forming box (1100");
F - optionally depositing in said second forming box (1100") a layer of second fibers from said first (1110') or said second (1110") fibrous material supply and optionally particulate material from a second particulate material supply (1710") onto said first fibrous web sub-sections (110'), whereby, supported by said second vacuum suction system (1500),
o whereby said blocked regions (1518) of said collection system (1500) remain essentially free of fibrous material,
thereby creating second fibrous web sections (110");
G - transferring said fibrous web sections (110) comprising
∘ at least said first sub-sections (110'), optionally comprising particulate material;
∘ optionally said second sub-sections (110"), optionally comprising particulate material;
∘ optionally particulate material (118) sandwiched between said first (110') and said second (110") web sub-sections
to said transfer unit (2100),
wherein said steps B to G are executed in the given order.

**4.** A process according to claim 3, wherein in steps B or F, the fibrous material supply is executed by individualizing said fibers from a supply in a fiber board form by a hammer mill, thereby preferably orienting the axis of the hammer mill cross-directionally.

**5.** A process according to any of claims 3 or 4, wherein in steps B or F, the fiber supply of the fibrous materials executed by enhancing the fiber flow by a venturi effect restriction in the fiber supply pipe.

**6.** A process according to any of claims 3 to 5, wherein in steps B or F, said depositing of said fibers is supported by a fiber distribution homogenization unit (1150) comprising multiple fiber homogenization tools (1153), preferably of the impeller type rotating around a vertical axis.

**7.** A process according to any of claims 3 to 6, wherein in step A1 said step of providing a first fibrous material supply (1110') and optionally a second fibrous material supply (1110") includes the step of providing untreated cellulose fibers.

**8.** A process for forming a laminate (210) comprising a series of web sections (110) according to any of claim 3 to 7, further comprising the steps of
A2 - further providing,
• a first envelope web (2312) from a first envelope web supply (2310) and a first envelope web combining roll (2315), preferably a vacuum roll;
o a second envelope web (2322) from as second envelope web supply (2320) and a second envelope web combining roll (2325);
∘ optionally a first (2410) and a second (2420) glue applicator unit, adapted to apply glue to said first (2312) or said second (2322) envelope web, respectively;
∘ optionally a final bonding roll (2430);
H - transferring said web sections (110) towards and through said nip of a first (2315) and a second (2325) combining roll;
I - transferring said first envelope web (2312) from said first envelope supply unit (2310) to said first envelope combining roll (2315), optionally applying thereby glue (2400') from said first glue applicator (2410);
K' - transferring said second envelope web (2322) to said second envelope web combining roll (2325), thereby optionally applying second glue (2400") from said second glue applicator (2420), and further into a nip between said second envelope web combining roll (2325) and said first envelope combining roll (2315);
L' - enveloping said series of web sections (110) in said gap between said combining roll (2315, 2325) in said first (2312) and second (2322) envelope web, thereby forming said laminate (210);
M - optionally transferring said laminate to a final bonding unit (2430), preferably a melt-fusion bonding unit.

**9.** A process of forming a laminate (210) according to claim 8, further comprising the steps of
A2 - further providing,
o transfer roll (2100);
o a second compaction unit (2200) comprising a nip formed between
□ a first roll, preferably a patterned roll (2210)
□ and a counteracting vacuum roll (2220);
H - transferring said web sections (110) to said transfer unit (2100) towards and through said nip of said second compaction unit (2200);
I - transferring said first envelope web (2312) from said first envelope supply unit (2310) to said first envelope combining roll (2315), optionally applying thereby a first glue (2400') from said first glue applicator (2410);
K - transferring said compressed web sections (110) to said first envelope combining roll (2315), thereby positioning it on said first envelope web (2312);
L - transferring said second envelope web (2322) to said second envelope web combining roll (2325), thereby optionally applying a second glue (2400") from said second glue applicator (2420), and further into a nip between said second envelope web combining roll (2325) and said first envelope combining roll (2315), thereby forming enveloped separated fibrous sections (100);
M - optionally transferring said enveloped separated sandwich web sections to a final bonding unit (2430), preferably a melt-fusion bonding unit;
N - forming individualized articles by finishing up said enveloped separated sandwich web sections by
∘ fully separating consecutive sandwich web sections from each other and/or packaging these enveloped separated fibrous web sections;
∘ packing it in roll-form with semi-separated separation lines between adjacent web sections,
wherein steps I to N are executed in the given order subsequent to step H.

**10.** A process for forming a laminate (210) comprising a series of web sections (110) according to any of claim 3 to 7, further comprising the steps of
A2 - further providing,
∘ a first envelope web (2312) from a first envelope web supply (2310) and optionally a first envelope web combining roll (2315);
∘ a second envelope web (2322) from a second web envelope web supply (2320) and a second envelope web combining roll (2325);
∘ optionally a first (2410) and a second (2420) glue applicator unit, adapted to apply glue to said first (2312) or said second envelope web (2322), respectively;
∘ optionally a final bonding roll (2430);
∘ a finishing unit, preferably comprising a separation unit and a packaging unit;
I - transferring said first envelope web from said first envelope supply unit (2310) to said first glue applicator (2410) and selectively apply glue (2400) to glued portions (2311) of said first envelope web (2312), separated by glue free portions (2313),
whereby said glued portions (2311) correspond in positioning to said air permeable regions (1512) of said collection system (1500), and preferably exhibit x-y-directionally size of at least 5 mm less than the air permeable regions (1512);
O - transferring said first envelope web (2312) towards said collection system (1500) at a speed corresponding to the speed of said collection system (1500) and positioning said glued portions (1311) over said air permeable regions (1512), preferably such that a glue free periphery of at least 5 mm relative to the air permeable portions (1512) is formed;
B' - depositing in deposition region (1103) of said first forming box (1100') a layer of first fibers of a first fibrous material from said first fibrous material supply (1110') and optionally particulate material from a first particulate material supply (1710') onto the first envelope web (2312) over said air permeable regions (1512) of said collection system (1500), whilst supporting the deposition by vacuum suction of said first vacuum suction system (1550') such that said blocked regions (1518) of said collection system (1550) are essentially free of fibrous material, thereby creating first fibrous web sub-sections (110');
C - transferring said first fibrous web sub-sections (110') to and through said first compaction unit (1600);
D - optionally transferring said first fibrous web sub-sections (110') to a further particulate material application system (1700‴) and applying particulate material onto the first fibrous web sub-sections (110'), thereby preferably leaving said blocked regions (1518) of said collection system (1500) free of particulate material, preferably supported by suction of a further third vacuum suction unit (1550‴);
E - optionally transferring said first fibrous web sub-sections (110') to and into said second forming box (1100");
F - optionally depositing in said second forming box (1100") a layer of second fibers from said first (1110') or said second (1110") fibrous material supply and optionally particulate material from a second particulate material supply (1710") onto said first fibrous web sub-sections (110'), whereby, supported by said second vacuum suction unit (1550"),
∘ said blocked regions (1518) of said collection system (1500) remain essentially free of fibrous material,
thereby creating second fibrous web sections (110");
G' - transferring said fibrous web sections (110) comprising
∘ at least said first sub-sections (110'), optionally comprising particulate material;
∘ optionally said second sub-sections (110"), optionally comprising particulate material;
∘ optionally particulate material (118) sandwiched between said first (110') and said second (110") web sub-sections
on said first envelope web (2312) to said second envelope web combining roll (2325);
K'- transferring said second envelope web (2322) to said second envelope web combining roll (2325), thereby optionally applying a second glue (2400") from said second glue applicator (2420);
L' - combining said series of web sections (110) on said first envelope web (2312) with said second envelope web (2322), thereby forming said laminate (210);
M - optionally transferring said laminate (210) to a final bonding unit (2430), preferably a melt-fusion bonding unit.

**11.** A process of forming a laminate (210) according to claim 10, further comprising the steps of
A2 - further providing
∘ transfer roll (2100);
∘ a second compaction unit (2200) comprising a nip formed between
▪ a first roll, preferably a patterned roll (2210)
▪ and a counteracting vacuum roll (2220);
H - transferring said web sections (110) on said first envelope web (2312) to said transfer unit (2100) towards and through said nip of said second compaction unit (2200), thereby forming a series of embossed web sections;
L - transferring said second envelope web (2322) to said second envelope web combining roll (2325), thereby optionally applying a glue (2400") from a glue applicator (2420), and combining it with said series of embossed web sections by said combining roll (2325), thereby forming enveloped separated fibrous sections (210);
M - optionally transferring said enveloped separated sandwich web sections to a final bonding unit (2430), preferably a melt-fusion bonding unit.

**11.** Forming a series of articles by
providing a laminate (210) according any of claims 8 to 10;
N1 - forming individualized articles by finishing up said laminate (210) comprising said enveloped separated sandwich web sections (110) by fully separating consecutive sandwich web sections from each other and/or packaging these enveloped separated fibrous structures,
or
N2 packing said laminate (210) in roll-form with semi-separated separation lines between adjacent enveloped separated sandwich web sections (110).
